# EUROPEAN PATENT SPECIFICATION

(11) **EP 2 579 937 B1**
(45) Date of publication and mention of the grant of the patent: **17.02.2021**
(21) Application number: 11793118.8
(22) Date of filing: 08.06.2011
(51) Int. Cl.: A61M 39/10, A61M 39/08

(54) **CONNECTOR ASSEMBLY**
VERBINDERBAUGRUPPE
ENSEMBLE RACCORD

(30) Priority: 02.02.2011 US 201161462405 P; 22.11.2010 US 458348 P; 17.08.2010 US 401671 P; 08.06.2010 US 397243 P
(43) Date of publication of application: 17.04.2013
(73) Proprietor: Yukon Medical, LLC, Durham, NC 27703 (US)
(72) Inventor: MOSLER, Theodore J., Durham NC 27703 (US); KOROGI, Todd M., Durham NC 27703 (US); FOSHEE, David L., Durham NC 27703 (US); PENNY, Matthew R., Durham NC 27703 (US); DICKEY, Mark S., Durham NC 27703 (US)
(74) Representative: Barker Brettell LLP
(86) International application number: PCT/US2011/039673
(87) International publication number: WO 2011/156521

(56) References cited:
- EP-A1- 2 153 864
- EP-A1- 2 153 864
- WO-A1-2006/103074
- US-A- 5 188 620
- US-A- 5 188 620
- US-A- 5 582 600
- US-A- 6 077 259
- US-A1- 2003 036 735
- US-A1- 2003 208 165
- US-A1- 2007 007 478
- US-A1- 2009 232 586
- US-A1- 2010 030 195

## Description

### TECHNICAL FIELD

The present disclosure relates generally to connectors and assemblies of connectors, specifically, connectors suitable for fluid transfer, for example, in the medical field of use.

### BACKGROUND

Using sharp needles to pierce a rubber septum of an IV or other fluid delivery device for administering medications presents a dangerous risk of accidental pricks from contaminated needles, causing a significant health risk to hospital personnel. Attempts to use blunt instruments to insert medication into the IV system have in the past resulted in unacceptable leakage problems.

US6077259 describes a contamination resistant connector for medical tubing that includes a first portion with a first fluid passage therethrough occluded by a covered normally closed first valve and a second portion with a second fluid passage therethrough. The second portion is conjugate to and releasably attachable to the first portion with the second fluid passage also being occluded by a covered normally closed second valve. The first valve and the second valve are both only uncovered and opened so that the first fluid passage and the second fluid passage are fluidly communicatively connected, and closed and covered by the respective selective conjugate engagement and disengagement of the first portion and the second portion.

WO2006/103074 describes a connector for medical application that includes fluidly connectable first and second flow pipes. The first flow pipe includes a connector skirt having a bayonet connection formation that includes an axial-locking surface and a radial-locking surface. The second flow pipe includes a cylindrical connector plug adapted for mating with the connector skirt. The connector plug includes a protrusion extending radially from the plug for being received by the bayonet connection formation of the skirt to lock the first pipe to the second pipe. When locked, adjacent surfaces of protrusion are in engagement with the axial-locking surface and the radial-locking surface to prevent unintentional axial disengagement and radial disengagement of the pipes. A spring presses corresponding surface of the protrusion against the axial-locking surface when the two pipes are connected.

US2009/232586 describes a sterile connector assembly for mounting on a fluid system that includes a first connector and a second connector. The first connector includes a stem defining a fluid passage therethrough, a first housing surrounding the stem and defining a first aperture, and a first valve disposed over the first aperture. The second connector includes a second housing configured to matingly engage the first housing. The second housing defines a second aperture and defines a seal structure. The seal structure is configured to engage the stem. The second connector also includes a second valve disposed over the second aperture. The second valve is configured to engage the first valve when the first housing engages the second housing.

### SUMMARY

Briefly, a connector assembly is provided comprising a cannula connector embodiment engagable with a septum connector embodiment, the assembly being capable of providing a locked configuration upon engagement of the connectors.

Thus, according to a first embodiment the claimed invention provides a cannula connector for establishing a locking connection with a septum connector, as defined in claim 1. The cannula connector comprises an access member having a generally annular flange disposed on one end and terminating in a cannula, the access member providing a generally cylindrical fluid passage between the annular flange and the cannula. A shroud comprising a first wall projects from the access member between the annular flange and the cannula, the first wall at least partially centrally surrounding the cannula forming an opening sized to receive a septum portion of the septum connector; a second wall and a third wall connecting a fourth wall, a least a portion of the fourth wall distally positioned from the first wall so as to provide a cavity in the opening. At least one aperture is formed through the fourth wall. In one aspect, at least a portion of the first wall can form an opening about the cannula that is generally cylindrical, the first wall can terminate in a generally annular edge. In other aspects alone or in combination, at least a portion of the cavity formed by the second, third, and fourth wall can be generally rectangular, arched, or square. The shroud can comprise apertures and/or protrusions for locking with corresponding protrusions and/or apertures of the septum connector.

According to a second embodiment the claimed invention provides a septum connector, as defined in claim 6. The septum connector is for establishing a locking configuration with the cannula connector of the first embodiment. The septum connector comprises a tubular housing comprising a first end and a second end along a longitudinal axis providing a generally cylindrical fluid passage between the first end and the second end. A penetrable septum is disposed on the first end sealing the first end. A cantilever is coupled to the housing and extends axially generally along the longitudinal axis of the tubular housing, the cantilever configured to deflect generally normal to the longitudinal axis of the housing and is configured to engage with the cannula connector. The penetrable septum can be a spilt septum. The cantilever can be coupled at one end either proximal to the penetrable septum or distal thereto, such that the terminus of the cantilever is either distal or proximal to the septum. The tubular housing can comprise apertures and/or protrusions for locking with corresponding protrusions and/or apertures of the shroud.

According to a third embodiment, the claimed invention provides a connector assembly as defined in claim 15. The connector assembly comprises the cannula connector of the first embodiment and the septum connector of the second embodiment.

### BRIEF DESCRIPTION OF THE DRAWINGS

In the following drawings, Figures 1A to 8G illustrate products that are described but not claimed. Figures 9A to 20F illustrate the claimed invention.
FIGs. 1A and 1B are a profile view and a sectional view along line 1B-1B, respectively, of an assembly in the engaged position as disclosed and described herein.
FIGs. 2A and 2B, are an exploded profile view and exploded sectional view along line 2B-2B, respectively, of the assembly of FIG. 1A, before connection;
FIGs. 2C and 2D are side and perspective views, respectively, of an exemplary penetrable septum as disclosed and described herein;
FIG. 2E: is a perspective view of the assembled connector of FIG. 2A;
FIGs. 3A, 3B, and 3C are a profile view, a sectional view along line 3B-3B, and a bottom view, respectively, of a connected assembly as disclosed and described herein;
FIGs. 4A, 4B, and 4C are an exploded side view, an exploded side view of the plane of the minor axis, and an exploded sectional view along line 4C-4C, respectively, of the assembly of FIGs. 3A, before connection;
FIGs. 5A, 5B, and 5C: are a profile view, a sectional view along line 5B-5B, and a bottom view, respectively, of a connected assembly as disclosed and described herein;
FIGs. 6A, 6B, and 6C: are an exploded side view, an exploded side view of the plane of the minor axis, and an exploded sectional view along line 6C-6C, respectively, of the assembly of FIG. 5A, before connection;
FIGs. 7A, 7B, 7C, 7D, and 7E are a side profile view, a perspective view, respectively, of a connected assembly, and an exploded first side view, an exploded second side view, and an exploded sectional view along line 7E-7E, respectively, of the assembly before connection, as disclosed and described herein;
FIGs. 8A, 8B, are a profile view and a sectional view along line 8B-8B, respectively, of a connected assembly as disclosed and described herein;
FIGs. 8C and 8D are perspective view of the assembly of FIG. 8A in an un-locked and locked configuration, respectively;
FIGs. 8E, 8F, and 8G are a first side view a second side view, and sectional view along line 8G-8G, respectively, of the assembly of FIG. 8A before connection;
FIG. 9A, 9B, 9C, 9D, 9E, and 9F are a first side view, a perspective view, and a second side view, respectively, of a connected assembly as disclosed and described herein, and two exploded side views, and an exploded sectional view, respectively, along line 9F-9F of the assembly of FIG. 9A before connection;
FIG. 10A is a bottom plan view of the septum connector of FIG. 9D;
FIG. 10B is a bottom plan view of the cannula connector of FIG. 9D;
FIG. 11A, 11B, 11C, 11D, 11E, and 11F are a first side view, a perspective view, and a second side view, respectively, of a connected assembly as disclosed and described herein, and two exploded side views, and an exploded sectional view, respectively, along line 11F-11F of the assembly of FIG. 11A before connection;
FIG. 12A, 12B, 12C, 12D, 12E, 12F, and 12G are a first side view, a second side view, and a third side view, respectively, of a connected assembly as disclosed and described herein, and two exploded side views, and an exploded sectional view, respectively, along line 12G-12G of the assembly of FIG. 12A before connection;
FIG. 13A, 13B, 13C, 13D, 13E, 13F, and 13G are a first side view, a second side view, and a third side view, respectively, of a connected assembly as disclosed and described herein, and two side views and a sectional view along line 13F-13F, and a detail sectional view, respectively, of the assembly of FIG. 13A before connection;
FIG. 14A, 14B, 14C, 14D, and 14E are a first side view, a second side view, a sectional view along line 14C-14C, a bottom view, and a top view, respectively, of the cannula connector of FIGs. 12A and 13A;
FIG. 15A, 15B, and 15C are a side view, an exploded side view, and an exploded sectional view along line 15C-15C, respectively, of a connected assembly as disclosed and described herein;
FIG. 16A, 16B, and 16C are a side view of a connected assembly as disclosed and described herein, and an exploded side view and an exploded sectional view along line 16C-16C, respectively, of the connected assembly of FIG. 16A;
FIG. 17A, 17B, and 17C, are an exploded profile view of a connected assembly as disclosed and described herein, and a bottom view and a top view of the assembly of FIG. 17A;
FIGs. 17D, 17E, and 17F are a side view, a sectional view along line 17E-17E, and a perspective view, respectively, of the connected assembly of FIG. 17A;
FIG. 18A, 18B, 18C, 18D, 18E, 18F, and 18G are a first side view, a second side view, and a sectional view along line 18C-18C, respectively, of a connected assembly as disclosed and described herein, and an exploded side view and perspective view, respectively, of the assembly of FIG. 18A;
FIG. 19A, 19B, 19C, 19D, 19E, and 19F are a first side view, a second side view, and a sectional view along line 19C-19C, respectively, of a connected assembly as disclosed and described herein, and an exploded first side view, exploded second side view, respectively, of the assembly of FIG. 19A, and a perspective view of FIG. 19A; and
FIG. 20A, 20B, 20C, 20D, 20E, and 20F are a first side view, a second side view, and a sectional view along line 20C-20C, respectively, of a connected assembly as disclosed and described herein, and an exploded profile view and exploded sectional view along line 20E-20E, respectively, of the assembly of FIG. 20A before connection, and a perspective view of the connected assembly of FIG. 20A.

### DETAILED DESCRIPTION

At least one solution provided by the connector assembly and/or connectors is to provide a reusable, cleanable, sealable and secure fluid path between an IV set and a fluid source container, such as a syringe, in order to transfer fluids into a patient. Pre-slit septum connectors herein disclosed will typically be attached to a patient's IV set. Immediately before use, the user or health care practitioner will wipe the pre-slit septum surface with an alcohol swab to disinfect the surface. The user will then push the cannula connector's cannula through the pre-slit septum to make a fluid path connection and engage the locking mechanism herein disclosed and described. After fluid transfer is complete, the locking mechanism is configured for disengagement and withdrawal of the cannula connecter. Disclosed and described is an inexpensive connector and/or assembly which can be provided sterile, and which requires specific or positive manipulation for disengagement of the assembly, rather than a disconnection by accidental handling or random or unintentional movements by the patient.

Embodiments of the present disclosure now will be described more fully hereinafter with reference to the accompanying drawings, in which embodiments of the disclosure are shown. This present disclosure may, however, be embodied in many different forms and should not be construed as limited to the embodiments set forth herein. Rather, these embodiments are provided so that this disclosure will be thorough and complete, and will fully convey the scope of the invention to those skilled in the art. Like numbers refer to like elements throughout. The precise shapes and sizes of the components herein described are not essential to the disclosure unless otherwise indicated. For ease of description, the connector assembly of this disclosure will be described in a normal (or typical) operating position and such terms as up, down, top, bottom, etc. will be used with reference to this position. It will be understood, however, that the connector assembly of this disclosure may be manufactured, stored, transported, used and sold in an orientation other than the position described.

Although such terms as first, second, etc. may be used herein to describe various elements, these elements should not be limited by these terms. These terms are only used to distinguish one element from another. For example, a first element could be termed a second element, and, similarly, a second element could be termed a first element. As used herein, the phrase "and/or" includes any and all combinations of one or more of the associated listed items.

When an element is referred to as being "on" or extending "onto" another element, it can be directly on or extend directly onto the other element or intervening elements may also be present. In contrast, when an element is referred to as being "directly on" or extending "directly onto" another element, there are no intervening elements present. When an element is referred to as being "connected" or "coupled" to another element, it can be directly connected or coupled to the other element or intervening elements may be present. In contrast, when an element is referred to as being "directly connected" or "directly coupled" to another element, there are no intervening elements present. It will be understood that these terms are intended to encompass different orientations of the element in addition to any orientation depicted in the figures.

Relative terms such as "below" or "above" or "upper" or "lower" or "horizontal" or "vertical" may be used herein to describe a relationship of one element to another element as illustrated in the figures. These terms are intended to encompass different orientations of the device in addition to the orientation depicted in the figures.

Relative terms such as "substantially" and "essentially" may be used herein to encompass, for example, manufacturing tolerances related to height, length, width, flatness, curvature, force, load, amount, relative orientation, etc. Such terms are used to describe an element or limitation with precision appropriate to the manufacture of such devices.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" "comprising," "includes" and/or "including" when used herein, specify the presence of stated features, integers, steps, operations, elements, and/or components, but do not preclude the presence or addition of one or more other features, integers, steps, operations, elements, components, and/or groups thereof.

In the figure descriptions, a gripping feature encompasses any of a number of surface effects including, but not limited to, embossed or debossed features, surface finishes and over-molded or two-shot features.

The embodiments described herein may be designed such that they are compatible and able to engage, but not necessarily lock, with standard and non-standard off-the-shelf syringes and luer connectors. This provides a potential cost reduction measure, since tubing or other components may be configured directly to the features of the embodiments herein disclosed, without the need for additional connecting components. The final assembly or connector components may also be affixed, releasably or integral with a syringe or other device. The embodiments described herein may alternately be equipped with other attachment features in place of the illustrated luer fittings, e.g., connectors for securing to various intravenous (IV) sets commercially available.

Housings of the connectors and the pre-slit septum are configured to provide unobstructed access to the critical interface surfaces to allow effective disinfection methodology, using, for example, the standard alcohol swabbing techniques. Housings and other edged surfaces can be configured with smooth, rounded surfaces and a low profile to minimize irritation to a patient's skin when present for extended periods of time on IV sets.

Unless otherwise defined, all terms used herein have the same meaning as commonly understood by one of ordinary skill in the art to which this invention belongs. Terms used herein should be interpreted as having a meaning that is consistent with their meaning in the context of this specification and the relevant art and will not be interpreted in an idealized or overly formal sense unless expressly so defined herein.

Numerous other advantages and features of the connector assembly as disclosed herein will become readily apparent from the claims, drawings and the detailed description of the disclosure.

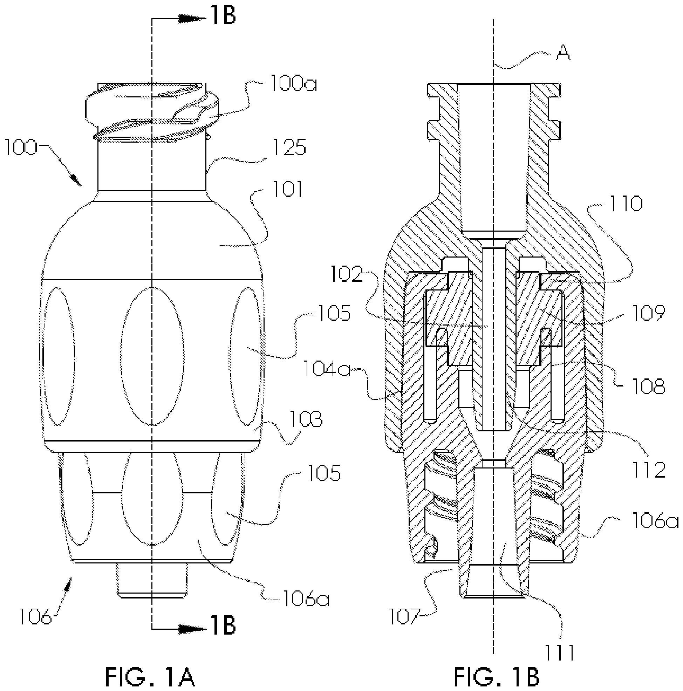

The claimed invention provides a cannula connector according to the first embodiment, a septum connector according to the second embodiment and a connector assembly according to the third embodiment. Figures 1A to 8G illustrate cannula connectors, septum connectors and assemblies thereof that are described but not claimed.

FIG. 1A and 1B show a connected assembly of cannula connector 100, having a longitudinal axis A, engaged with septum connector 106 in a profile view and sectional view along line 1B-1B. In this configuration, cannula connector 100 provides a taper lock engagement with septum connector 106. Cannula connector 100 comprises a generally annular shroud 101 disposed about blunt cannula 112 forming a cavity between the shroud and blunt cannula. Neck 125 extends outwardly about the shroud 101 along longitudinal axis A and comprises threaded female luer 100a providing fluid communication with lumen 102 forming a fluid path generally along axis A.

Septum connector 106 comprises a housing 106a extending along axis A having at one end a skirt generally surrounding male luer 107 with lumen 111. Housing 106a includes internal housing seat 108 configured to receive pre-slit septum 109 for presentation at an opposite end of housing 106a, which is secured to housing seat 108 by rim 110. Septum 109 can be configured in housing seat 108 uncompressed or held in compression by rim 110. Septum connector 106 comprises a matching conical taper to that of cannula connector 100 to provide a taper locking interference 104a when engaging cannula component along axis A. Gripping features 105 provide a surface for engaging and disengaging the connectors 100 and 106 of the assembly. The proximal opening of lumen 111 may be sized to receive IV tubing or other alternative fluid delivery device couplings. Cannula connector 100 provides satisfactory leak pressure after multiple connections and disconnections to septum connector 106 and long periods of indwell of the blunt cannula.

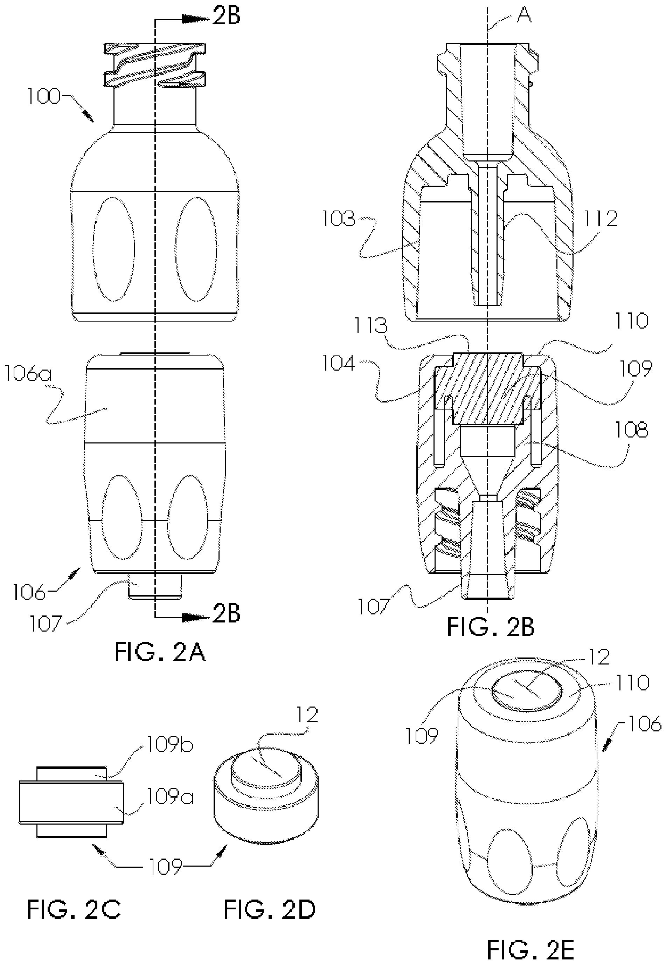

FIG. 2A, 2B, and 2E show a profile view, perspective view, and sectional view along line 2B-2B of the pre-engaged connectors 100 and 106, interior wall 103 of shroud is shown having a shallow conical taper that interfaces with the exterior wall 104 of the septum housing 106a.

Referring to FIGS. 2C and 2D, pre-slit septum 109 is shown in side and perspective view, respectively, having a central portion 109a with symmetrical smaller top and bottom portions 109b to allow symmetrical assembly to housing seat 108. Slit 12 allows cannula 112 to pass through on insertion. Exposed surface 113 of pre-slit septum 109 can be smooth and unobstructed, and can be flush or slightly raised, to provide an accessible surface for swabbing with disinfectant prior to engagement or thereafter. As an alternate to forming the slit 12 completely through the septum 109, a slit 12 can be formed only partly through the septum 109, so that until used for the first time, the septum 109 provides complete sealing of the end it is assembled to. The septum 109 can alternatively be formed in two parts, with one part having a slit extending entirely therethrough. A second part can be formed without a slit, the two parts located adjacent one another in the access site of the housing. The slit 12 can be wider at the exposed surface of the septum than at the bottom for aiding blunt cannula alignment with the slit upon engaging the assembly. While slit 12 dimensions can be configured for the particular end-use application (or blunt cannula diameter), slit could have a length with a range on the order of 0.03 inches (0.762 centimeters) to 0.150 inches (0.381 centimeters). By way of example, a slit length on the order of 0.07 inches (0.1778 centimeters) will be used in combination with a blunt cannula having a diameter on the order of 0.1 inches (0.254 centimeters). When initially used, blunt cannula 112 of the cannula connector 100 will be forced through the slit 12, providing access for the blunt cannula to lumen 111, of septum connector 106 and thus providing for fluid communication between access member 125 and lumen 111. In other aspects, septum configured as shown in FIG. 2E presents an outwardly extending curved exterior surface, slightly raised above the plane of the rim 110 to facilitate swapping and avoiding "pooling" of the disinfectant at the interface of rim 110 with the septum.

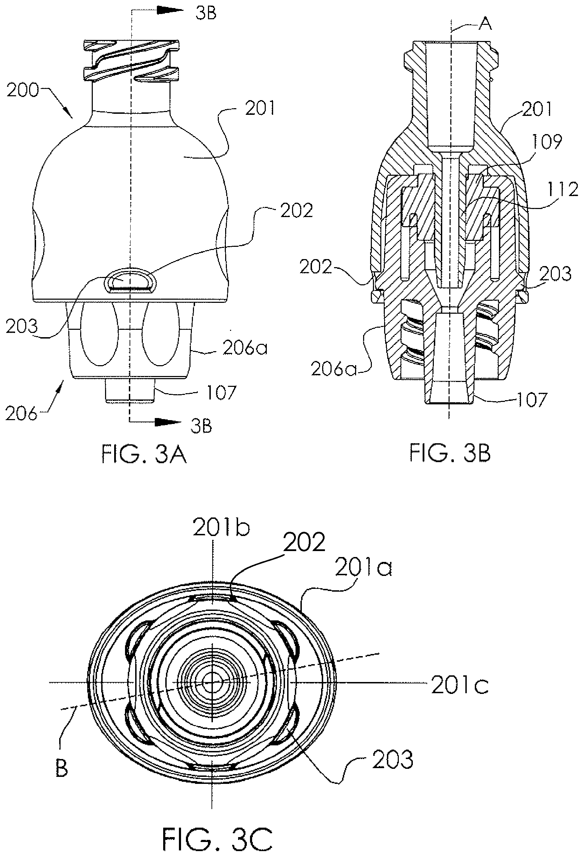

FIG. 3A, 3B, and 3C show a profile view and sectional view along line 3B-3B, and a bottom view, respectively, of cannula connector 200 and pre-slit septum connector 206, in an alternative locking engagement configuration. In this configuration, shroud 201 of cannula connector 200 has a generally elliptical cross section 201a, having a length along major axis 201c slightly greater than the width along minor axis 201b (normal to major axis 201c). As shown, a pair of spaced-apart apertures 202 are positioned along minor axis 201b of shroud 201. Septum connector 206 has a plurality of spaced-apart protrusions 203, on the outer surface of connector 206 between septum 109 and skirt 206a having threaded interior circumferential housing, the protrusions configured to engage apertures 202 of cannula connector 200. When cannula connector 200 is engaged with septum connector 206, shroud 201 flexes outwardly to allow protrusions 203 to be received in apertures 202, providing a locking interface. To detach cannula connector 200, a user exerts a force on the major axis 201c of shroud 201, causing it to deform outwardly about minor axis 201b, whereas apertures 202 extend along axis B beyond protrusions 203 so that the connectors are movable along axis A for disconnection. Protrusions 203 and apertures 202 can be of any shape, such as rectangular, square, oval, or dome-like as shown and preferably are arranged annularly about axis B. Protrusions 203 are generally configured to have a sloping shoulder on an upper surface and a generally straight edge on a bottom surface to provide for ease of engagement/disengagement.

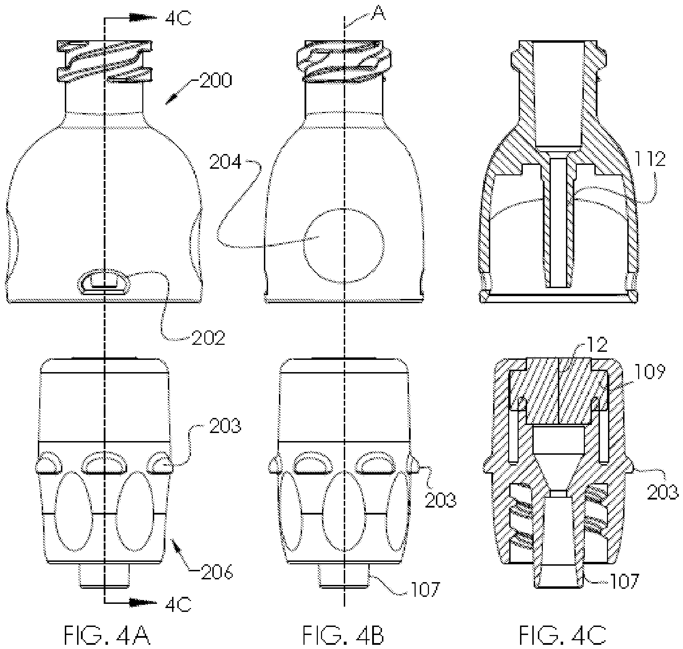

FIGs. 4A, 4B, and 4C show a profile view of the plane of major axis 201c and a projected view of the plane of minor axis 201b, and a sectional view along line 4C-4C, respectively, of the assembly of FIG. 3A before locking engagement. Grip features 204 can be configured to guide user as to where the user should squeeze the shroud to disengage the locking features provided by protrusions 203 and aperture 202.

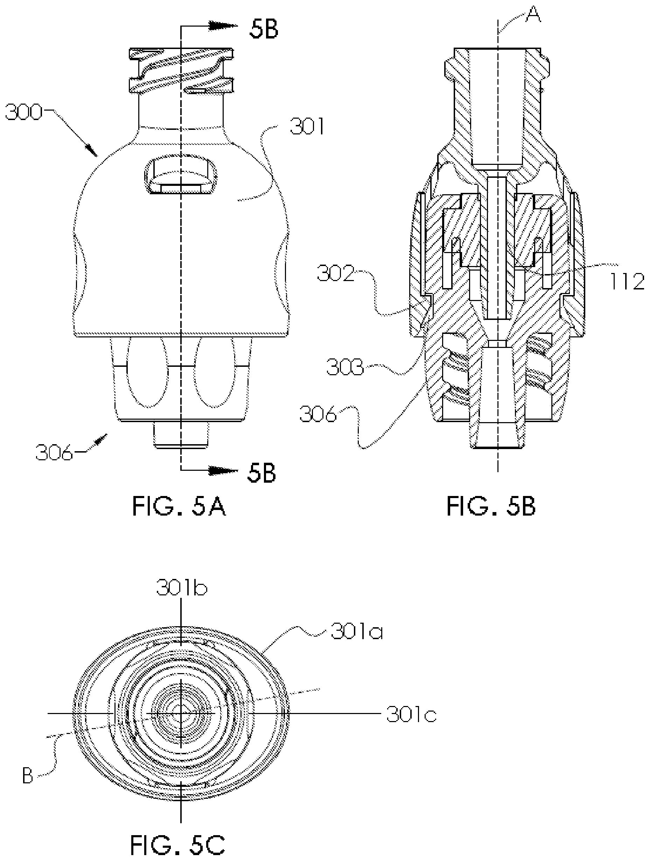

FIG. 5A, 5B, and 5C present an alternative locking configuration of the assembly of FIG. 3A, whereas the protrusions and apertures are generally reversed about the septum and cannula connector. Thus, a profile view, a sectional view along line 5B-5B, and a bottom view of the engaged assembly with an alternative locking configuration is shown where in this alternate configuration, shroud 301 of cannula connector 300 has an elliptical cross sectional 301a with protrusions 302 on its minor axis 301b. These protrusions cooperatively engage apertures 303 on the outside perimeter of septum connector 306. When cannula connector 300 engages septum connector 306, shroud 301 flexes out to allow protrusions 302 to snap into the apertures 303, providing a locking configuration. To disengage the assembly of connectors 300, 306, the user can press on major axis 301c of shroud 301, causing it to deform along the minor axis 301b until protrusions 302 are free of apertures 303 allowing disengagement of the assembly. As shown, protrusions 302 are generally configured to have an angled shoulder facing each other and a generally straight edge on a top surface, whereas, apertures 303 are generally configured to have a sloping shoulder on a bottom surface and a generally straight edge on a top surface to provide for ease of engagement/disengagement. Other configurations of protrusions and cooperative apertures can be used.

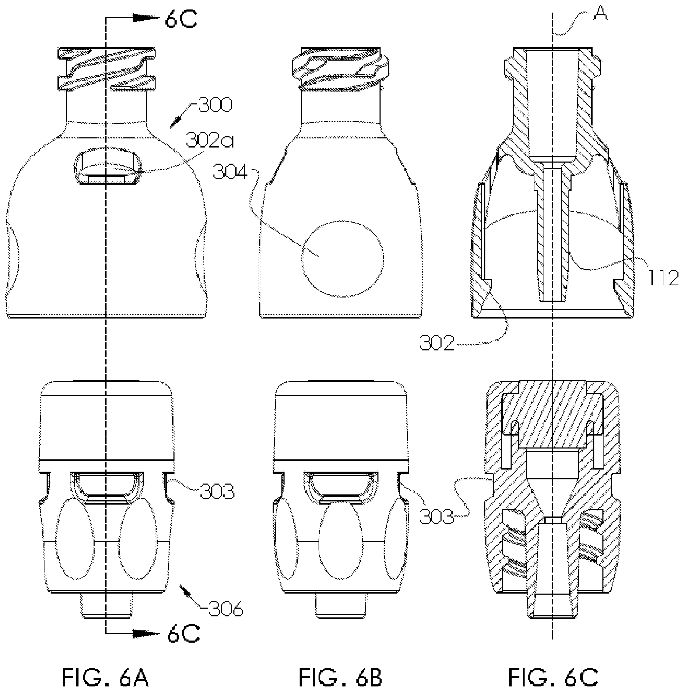

FIG. 6A, 6B, and 6C show a profile view of the plane of the major axis with sectional plane, a projected view of the plane of the minor axis, and a sectional view along line 6C-6C of the connectors of FIG. 5A before connection. FIG. 6A shows undercut 302a in line with protrusion 302 provided in shroud 301 for assisted release of connector 300 during molding. Grip features 304 of the cannula shroud indicate where the user should squeeze the shroud to unlock the snap feature as constituted by protrusions 302 and apertures 303.

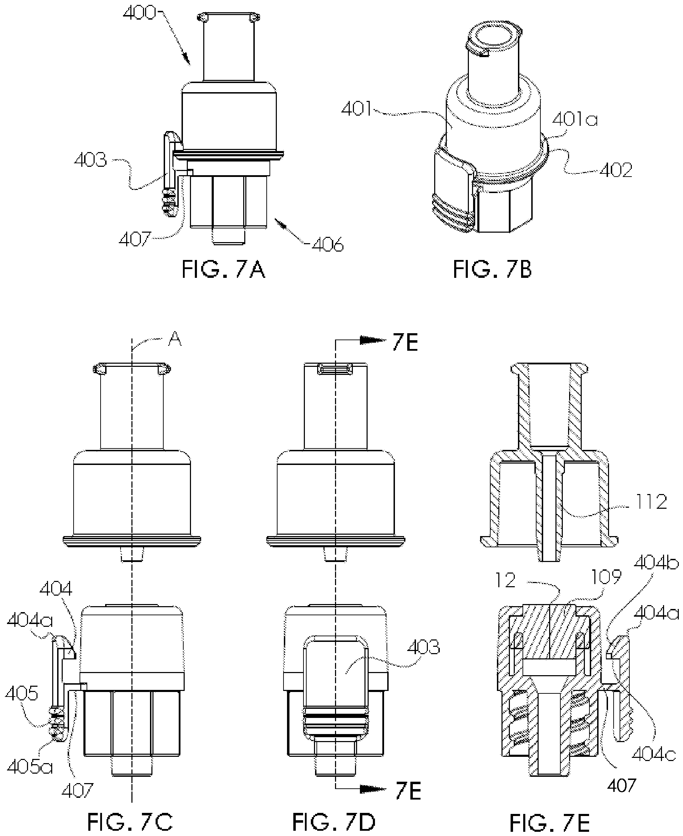

FIGs. 7A and 7B show a profile and a perspective view of a connected assembly, respectfully, in a locked configuration. FIGs. 7C, 7D, and 7E show a first side view, a second side view rotated about 90 degrees from the first side view, and a sectional view along line 7E-7E, respectively, of the assembly of FIG. 7A before connection. In this configuration, cannula connector 400 has a generally cylindrical shroud 401 with a generally annular rim 402 projecting outwardly from edge 401a of shroud 401 sized and configured for engaging locking means of septum connector 406 discussed below. Rim 402 projects outwardly essentially normal to longitudinal axis A. Rim 402 can be tapered, as shown, having a flat top surface and inwardly angled bottom surface. Septum connector 406 comprises cooperating flexible cantilever 403, which is coupled to outer surface of connector 406 via feature 407. Feature 407 projects from connector 406 essentially normal to longitudinal axis A of connector 406 to support cantilever 403. Longitudinal axis of cantilever 403 is generally parallel to longitudinal axis A of connector 406. Cantilever 403 comprises proximal end 404a and distal end 405a oppositely positioned about feature 407. Proximal end 404a includes inwardly facing tab 404 defined by an undercut feature generally configured with angled shoulder 404b downwardly facing inward towards connector 406 so as to cooperatively engage rim 402 upon engagement. Generally straight edge 404c on the bottom surface of tab 404, secures rim 402 and can allow the connectors to rotate after locking. Distal end 405a comprises grip features 405, which can indicate where the tab can be depressed to disengage cannula connector 400 from septum connector 406.

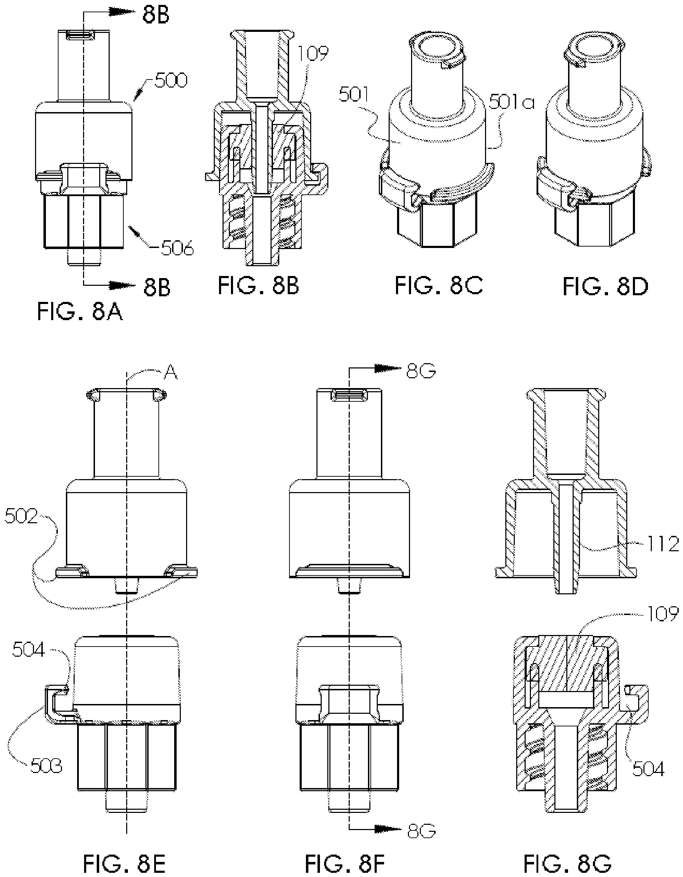

FIGs. 8A, 8B, 8C, and 8D show a profile view, a sectional view along line 8B-8B, and perspective views, respectively, of a connected assembly comprising cannula connector 500 and septum connector 506. This configuration is similar in design to that of FIGs. 7A-7E, except the assembly is designed for "twist-locking" between the connectors, described as follows. In this alternate configuration, cannula connector 500 has a generally cylindrical shroud 501 having a segmented rim projecting outwardly from distal edge 501a, providing a plurality of spaced-apart, sections 502 on distal edge 501a. FIGs. 8E, 8F, and 8G show first side view and a second side view rotated about 90 degrees, and a sectional view along line 8G-8G of the assembly in a pre-locked configuration. A rigid, C-shaped boss 503 protrudes outwardly from the outer surface of septum connector 506, the boss geometry creating a retention cavity 504 configured to receive sections 502 in the segmented rim upon alignment of the boss. Blunt cannula 112 is shown fully penetrating pre-slit septum 109 in FIG. 8B, and upon twisting of either connector about 90 degrees about the longitudinal axis A, provides for locking of the assembly. In one aspect, there can be a screw thread engagement between the sections 502 of cannula connector 500 and the boss 503 of septum connector 506 such that there is an axial displacement force of cannula connector away from the connector 506 upon rotating from its engaged state to its disengaged state, which can also be supplemented by the ejection force asserted by the septum on the penetrated cannula. Penetrable septum 109, as shown in FIG. 8G, has an exposed upper surface flush with the first end of the tubular housing along the longitudinal axis, however, in a preferred configuration, septum 109 extends beyond the first end of the tubular housing along the longitudinal axis, the upper surface being configured and shaped for disinfection by wiping, for example, with an alcohol swab.

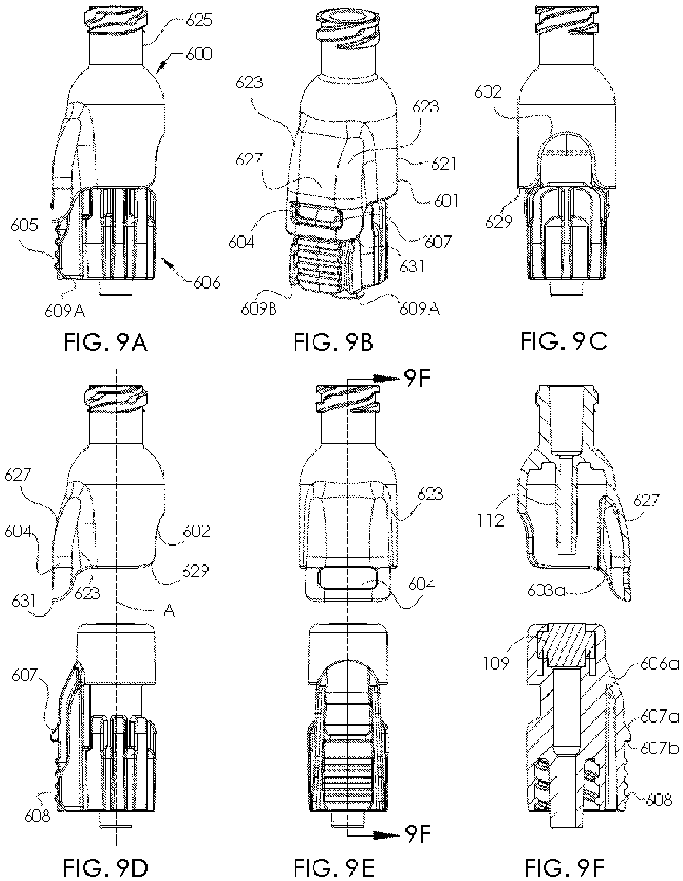

FIG. 9A, 9B, and 9C show a first side view, a perspective view, and a second side view rotated about 90 degrees from the first side view, respectively, of a connected assembly comprising cannula connector 600 and septum connector 606. FIGs. 9D, 9E, and 9F show a first exploded view, a second exploded view rotated about 90 degrees from the first view, and an exploded sectional view along line 9F-9F of cannula connector 600 and septum connector 606 prior to engagement. In this configuration, cannula connector 600 has a shroud 601 projecting downwardly from access member 625 (e.g., luer with annular, threaded flange), shroud 601 comprising a first wall 621 terminating, as shown, in a generally annular shaped edge 629, wall 621 generally sized and configured to receive septum 109 and septum connector 606. Wall 621 is adjacent to a second wall and a third wall 623, orientated generally parallel to longitudinal axis A and can taper towards access member 625, as shown, so as to provide a continuously sloping appearance to the shroud exterior and ease of manufacture. Walls 623 are joined by fourth wall 627 forming a cavity 603a distally positioned away from wall 621 of shroud 601. Third wall 627 extends beyond edge 629 of wall 621 to edge 631 at one end and merges with shroud wall 621 near access member 625. Wall 627 has aperture 604 positioned in proximity to edge 631 for receiving tab 607 of septum connector 606. Wall 621 also has cut-away 602, shown as arch-like feature, along distal edge 629 of shroud 600. Cut-away 602 is positioned in wall 621 generally 180 degrees from aperture 604 and is configured to receive a tubular portion (e.g., of a Y- or T- septum connector, or other device) associated with septum connector 606. Aperture 604 passes through wall 627 accessing cavity 603a. Cavity 603a can have any taper or be squared off in proximity to access member 625. Access member can be a female luer as shown or other adapter.

Septum connector 606 comprises cantilever 605, its proximal end 606a coupled to the outer surface of connector 606, in proximity to septum 109, and projecting generally downwardly and parallel to longitudinal axis A. Cantilever 605 has tab 607 projecting outwardly from the level and away from connector 606. Tab 607 is configured to cooperatively engage aperture 604 of cannula connector 600 during engagement, the tab having a generally sloping shoulder on a top surface 607a and a generally straight edge 607b on a bottom surface thereof to provide for ease of engagement/disengagement. Cantilever 605 and tab 607 are configured so as to be received by aperture 604 of shroud 601 upon essentially complete penetration of blunt cannula 112 thru septum 109, providing a locking configuration. Deflecting cantilever 605 towards septum connector 606 release tab 607 from aperture 604 for disengagement of the connected assembly. Cantilever 605 can include grip or tactile features 608, indicating where it can be depressed to release cannula connector for disengagement. Along one or both sides of cantilever 605, rib features 609A and 609B which project outwardly from connector 606 and essentially parallel to each other along the longitudinal axis A, configured to cooperatively engage cavity 603a of cannula connector 600, can be provided so as to prevent the cantilever or tab from snagging on its surroundings.

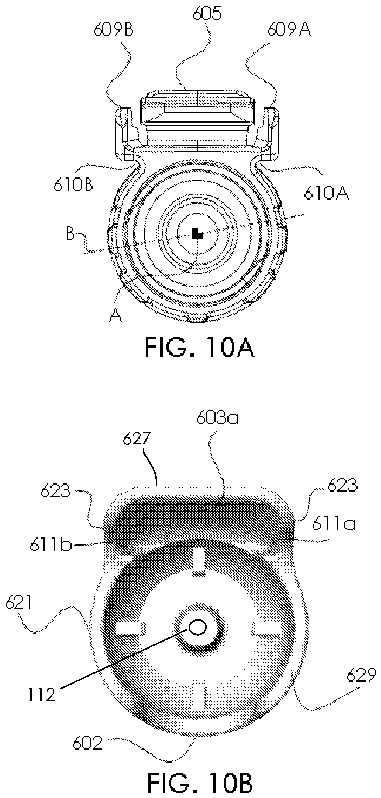

FIG. 10A shows a bottom view of septum connector 606 having distal end of cantilever 605 shown spaced away from outer surface of the connector so as to provide for deflection of tab 607. FIG. 10B shows bottom view of cannula connector 600 having second and third walls 623 and fourth wall 627 forming a generally arched shaped cavity 603a for receiving cantilever 605 of septum connector 606 for locking the connectors. Cavity 603a can be of other geometric shapes, such as generally a square, or generally rectangular. Cavity 603a preferably tapers towards the access member 625 of connector 600 so as to act as a stop for the movement of the septum connector and/or present a continuous shroud-like appearance to the outer perimeter of the cannula connector. However, the arrangement of the walls 623 and 627 can be of an alternate geometric design, or include other walls to provide a box-like or squared off appearance. Likewise, cut-away 602 can be of alternate shape. Septum connector 606 comprises recess 610A and 610B, positioned generally beneath each protective rib feature 609A and 609B. Recesses 610A, 610B are configured to cooperatively engage shelves 611A and 611B on the interior of cannula connector 600. The combination of recess 610A, 610B and shelf features 611A, 611B substantially prevent cantilever 605 and/or tab 607 from being pulled away from aperture 604 on cannula connector 600 after engagement of the connectors, thus preventing accidental disengagement.

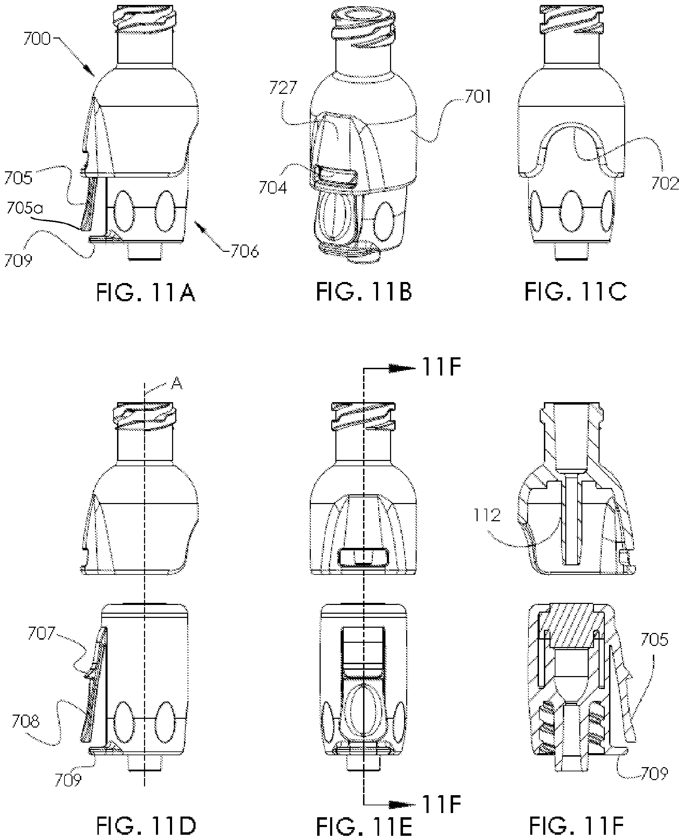

FIG. 11A, 11B, and 11C show a first side view, a perspective view, and a second side view rotated about 90 degrees from the first side view, respectively, of a connected assembly of cannula connector 700 with septum connector 706. FIGs. 11D, 11E, and 11F show a first side exploded view, a second side exploded view rotated about 90 degrees from the first side view, and a sectional view along line 11F-11F, respectively, of said assembly prior to engagement. In this configuration, cannula connector 700 (or 600) has similar features to that described above for connector 600. Similar to the septum connector 606, connector 706 comprises cantilever 705, its proximal end 705a coupled to the outer surface of connector 706, in proximity to septum 109, and projecting generally downwardly and parallel to longitudinal axis A. Septum connector differs from connector 606 in that a single rib 709 projects outwardly, generally normal to the longitudinal axis A, from perimeter outer surface of connector 706 at distal end 706a, just below distal end 705a of cantilever 705, so as to substantially prevent cantilever 705 from catching on its surroundings or accidentally acutuating. Cantilever 705 can include grip or tactile features 608, indicating where it can be depressed/deflected towards septum connector 706 to disengage cannula connector.

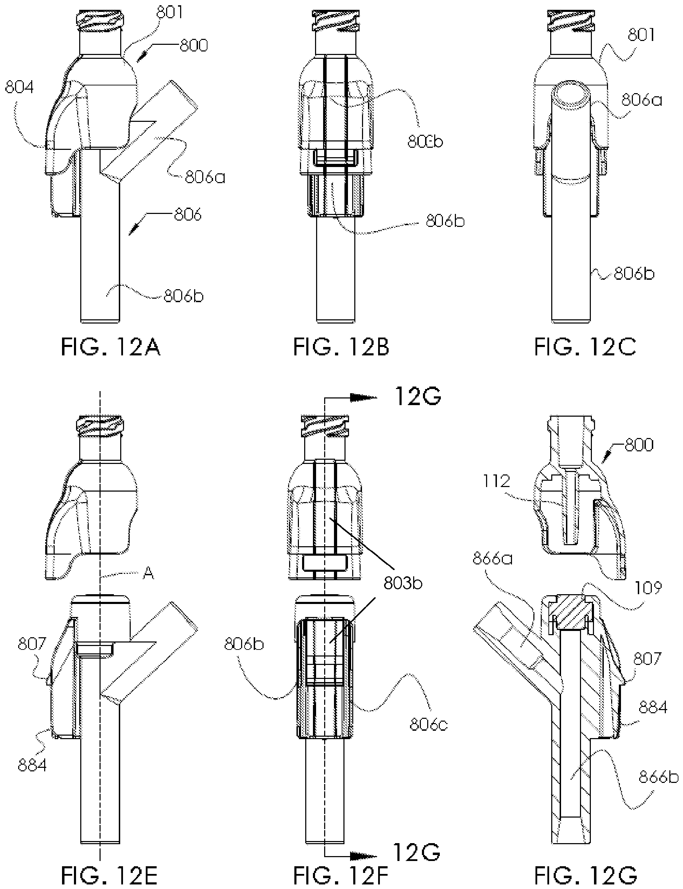

FIG. 12A, 12B, and 12C show a first side view, a second side view rotated about 90 degrees, and a third view rotated an additional 90 degrees, respectively, of an assembly comprising cannula connector 800, similar to that of connector 600 above, except as to textured feature 803b on wall 827 of shroud 801 and Y-connector 806 having engaging feature similar to that of connector 606 of FIG. 9F. FIGs. 12D, 12E, and 12F show a first side exploded view, a second side exploded view rotated about 90 degrees, and a sectional view along line 12G-12G, respectively, of said assembly prior to engagement. In this configuration, cannula connector 800 (600) is mated with Y-connector 806 having tube portions 806a, 806b with corresponding fluid channels 866a and 866b that can attach IV tubing (not shown), for example. When assembled into a tubing system, Y-connector 806 permits injection and withdrawals of fluids through the pre-slit septum 109. As previously described, a pair of integrated ribs 806b and 806c protect cantilever 884 and/or tab 807 from being inadvertently pressed upon or caught on loose tubing. Alignable textured and/or colored strips 803b can provide for gripping and/or a visual reference for aligning the two components during connection.

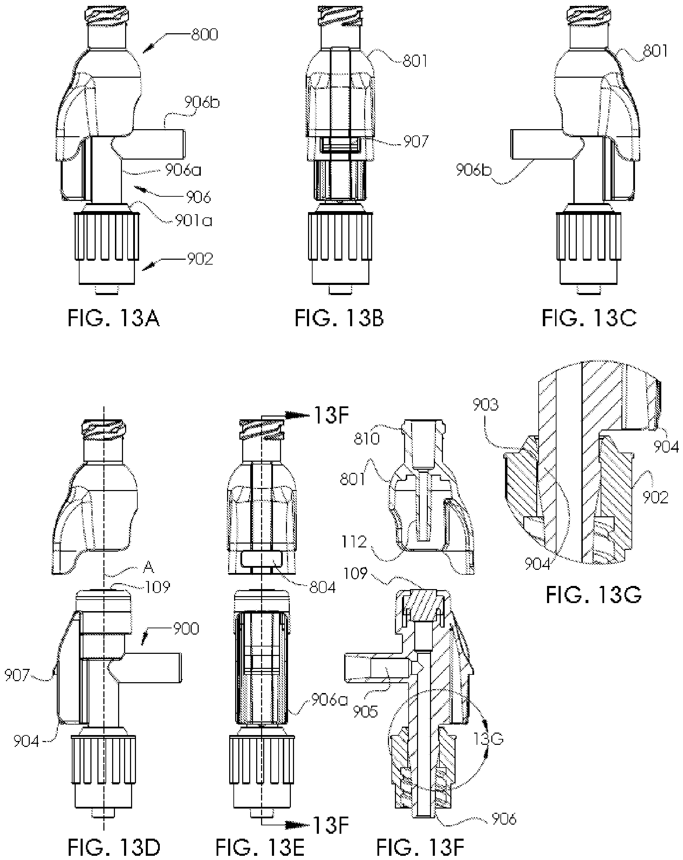

FIGs. 13A, 13B, and 13C show a first side view, a second side view, and a third side view, respectively, of a connected assembly of cannula connector 800 (of FIG. 12A) and T-site septum connector 900. FIGs. 13D, 13E, 13F, and 13G two side views and a sectional view along line 13F-13F, and a detail sectional view, respectively, of the assembly of FIG. 13A before connection prior to engagement. In this configuration, cannula connector 800 is mated with a T-site septum connector 900 having cantilever 904 and tab 907 similar to that of connector 806. T-site connector 900 comprises tube portions 906a, 906b, male luer collar 902 projecting downwardly from tube portion 906a, and a pre-slit septum 109 at the opposing end of portion 906a. With reference to FIG. 13G, as assembled, externally positioned annular shelf 903 of tube portion 906a cooperatively interferes with internally positioned annular shelf 914 on male collar 902 to keep the two components attached while permitting the collar to spin freely. When assembled into a tubing system, the T-site assembly of FIG. 13A permits injection and withdrawals of fluids through the pre-slit septum 109 in a configuration most commonly used with IV extension sets. Spinning lock collars can also be configured on the septum connector aspects disclosed herein such that the spin collar is molded together with the male luer feature, thus making a rigid male luer connection.

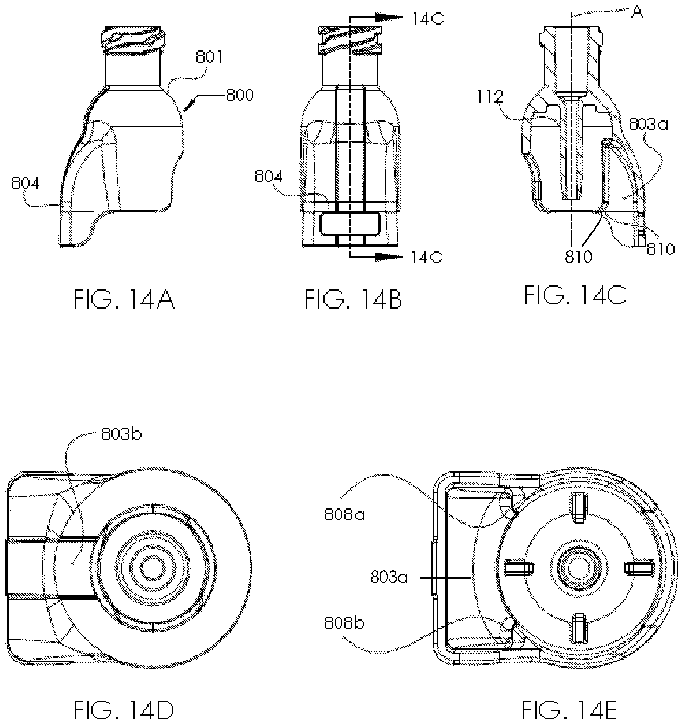

FIG. 14A, 14B, 14C, 14D, and 14E show a first side view, a second side view, a sectional view along line 14C-14C, a bottom view, and a top view, respectively, of cannula connector 800. Cannula connector 800 can include shelf features 808a and 808b (FIG. 14E) on the interior of cannula connector proximal to walls of cavity 803a to prevent a tab from a septum connector from being pulled away from aperture 804. A portion of distal edge 801a of shroud 801 contains a chamfer 810 to provide for ease of insertion of the cantilever of the septum connector during locking engagement of the connectors. Cannula connectors 600, 700, 800, and those disclosed below (1000, 1200) are adapted to securely engage any commercially available septum connector regardless of whether the septum connector contains locking features. Likewise, the septum connectors disclosed and described herein, regardless of the locking feature, can be used with other commercially available cannula connectors, for example, InterLink connector (Baxter, Deerfield, IL) and the like.

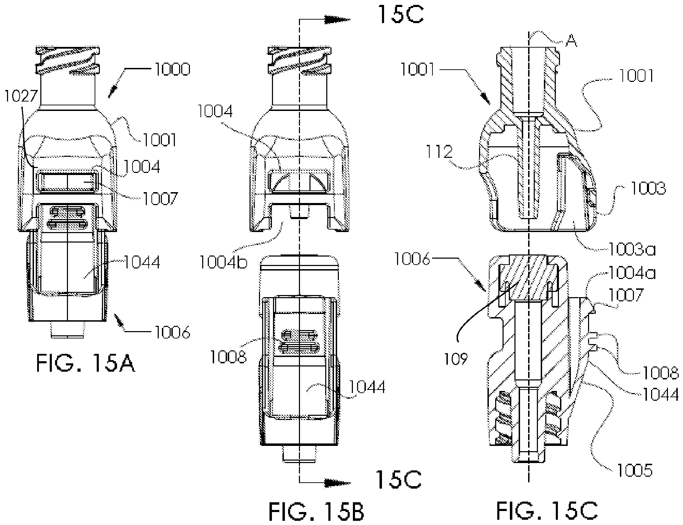

FIG. 15A, 15B, and 15C show are a side view, an exploded side view, and an exploded sectional view along line 15C-15C, respectively, of cannula connector 1000 and septum connector 1006. In this configuration, cannula connector 1000 has a shroud 1001 generally similar to that of connector 600 with cut-away 1004b and aperture 1004 providing access to cavity 1003a. Aperture 1004 in wall 1027 is positioned over second cut-away 1004b. Cantilever 1044 of septum connector 1006 is configured essentially reverse to connector 606 (of FIG. 9A) in that cantilever 1044 is connected in proximity to distal end 1005 of connector 1006 and away from septum 109 such that terminus 1004a of cantilever is positioned proximal to septum. Tab 1007 has a generally sloping shoulder on a top surface and a generally straight edge on a bottom surface (as described above) that is received by cavity 1003a and aperture 1004 during engagement of the connectors. Tab 1007 includes grip features 1008, indicating where the tab can be depressed to release cannula connector for detachment. Additional cut-away 1004b of connector 1000 provides more surface area of terminus 1004a of cantilever 1044 to access the cantilever.

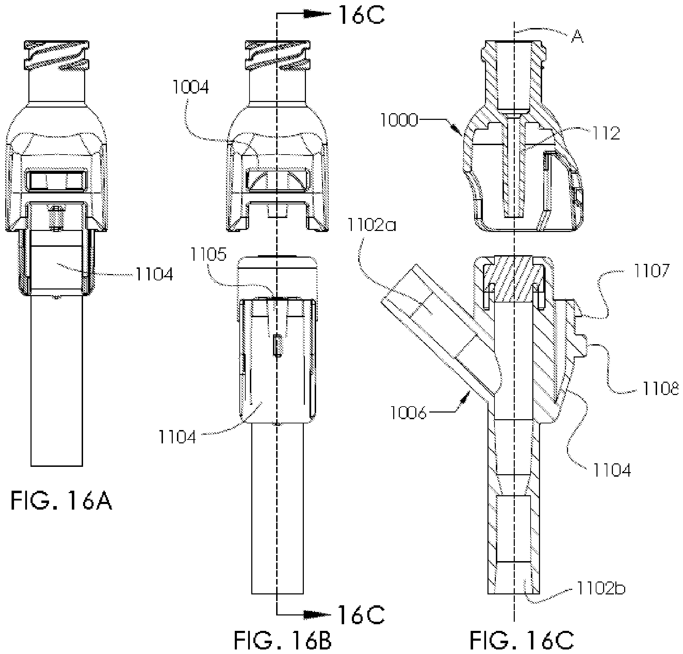

FIG. 16A shows a first side view of a connected assembly comprising cannula connector 1000 (as previously described above) and Y-connector 1106. FIGs. 16B and 16C show an exploded first side view and an exploded sectional view along line 16C-16C of connectors 1000, 1106. In this configuration, cannula connector 1000 is configured for engagement with Y-connector 1106 comprising two inner fluid channels 1102a and 1102b, e.g., that permit attachment of IV tubing (not shown). Y-connector 1106 includes cantilever 1104 and tab 1107 essentially the same as described above, with grip feature 1108. It is self-evident this aspect or the septum connector in combination with cannula connector 1000 can be integrated with other locking connectors such as the T-site component 900.

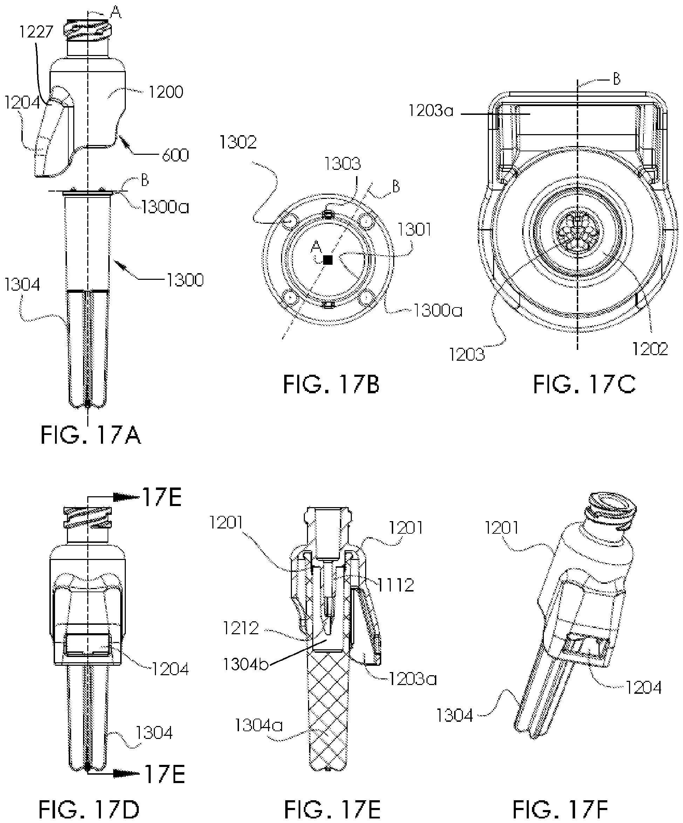

FIG. 17A is an exploded view of cannula connector 1200 and cannula cover 1300, suitable for sterilization by high energy radiation or ethylene oxide and for maintaining sterility of the connector. Connector 1200 is similar to connectors 600, 700, and 800, but for a side-ported cannula described below. Connector 1200 comprises shroud 1201 generally similar to that of connector 600, having wall 1227 and aperture 1204 providing access to tapered cavity 1203a, similar to cannula connectors 600, 1000 but for a side-vented blunt cannula 1112. FIG. 17B is a bottom view of cover 1300 having proximal end 1300a with internal surface 1301 and space-apart protrusions 1302 positioned about proximal end 1300a annularly about longitudinal axis A. Vent channels 1303 positioned annularly about longitudinal axis A provide a flow path for sterilization. FIGs. 17C, 17D, 17E, and 17F show a bottom view, a front view, a sectional view along line 17E-17E, and a perspective view of said assembled device. In this configuration, cannula connector 1200 is configured to engage with protective spike cover 1300, where a boss feature 1202 provides an interference fit with internal cover surface 1301 such that cover 1300 does not engage aperture 1204. Blunt cannula 1112 contains one or more slits 1212 generally parallel with longitudinal axis A creating an indirect flow path during fluid transfer ("side-ported"). Cover 1300 has solid body 1304a with space 1304b sized to accommodate cannula 112. Cover 1300 can be removed by grasping the grip features 1304 of cover and pulling downwardly.

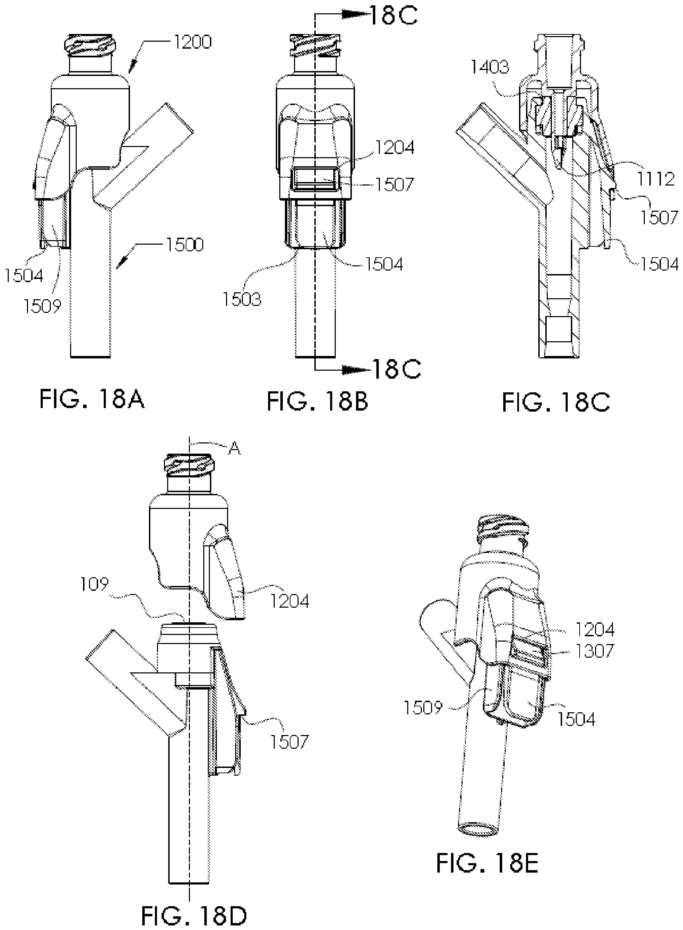

FIGs. 18A, 18B, 18C, 18D, 18E, 18F, and 18G are similar to aspects disclosed in FIGs 12A-12G but for cantilever 1504 and/or bracketing ribs 1509 and/or tab 1507 having rounded edges 1502 to provide access for engaging and disengaging cannula connector 1200 from Y-connector 1500 and to prevent snagging of the connectors during use.

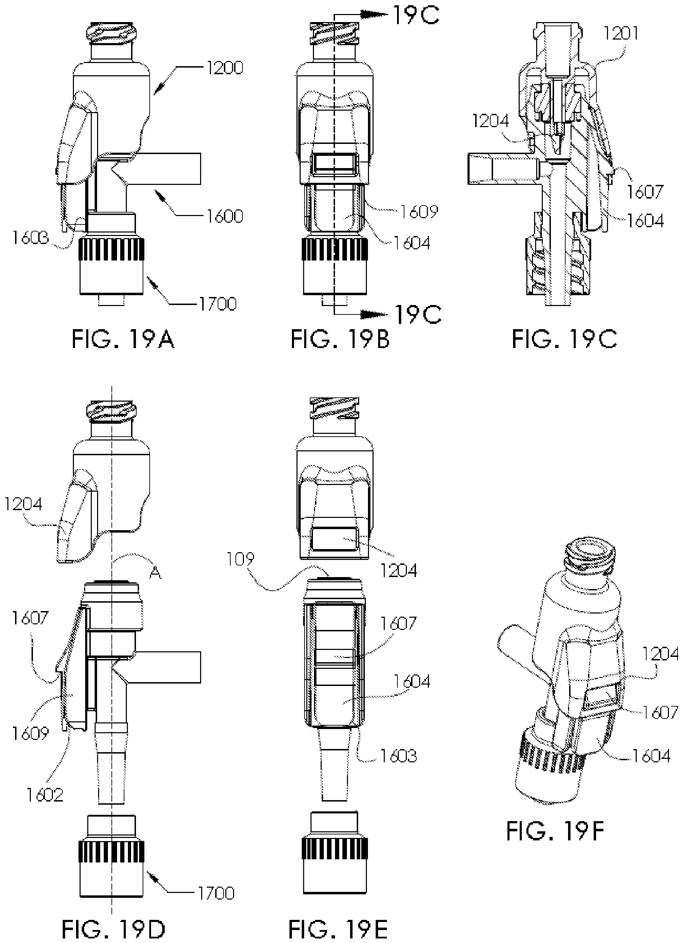

Likewise, FIG. 19A, 19B, 19C, 19D, 19E, and 19F are similar to aspects disclosed in FIGs 13A-13G but for cantilever 1604 and/or bracketing ribs 1609 and/or tab 1607 having rounded edges 1603 to provide access for engaging and disengaging cannula connector 1200 from T-site septum connector 1600. Freely revolving threaded lock collar 1700 is provided for securing the septum connector 1600 to other devices via a thread connection.

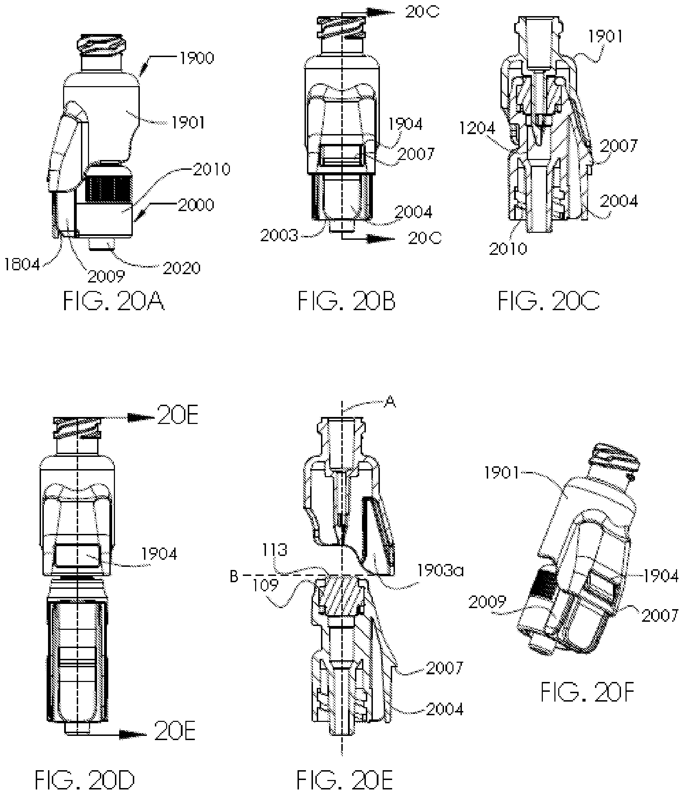

FIG. 20A, 20B, 20C, and 20F show a first side view, a second side view rotated about 90 degrees, and a sectional view along line 20C-20C, respectively, of a connected assembly comprising cannula connector 1900 and septum connector 2000. FIGs. 20D, 20E, and 20F show an exploded side view, and sectional view along line 20E-20E of said connectors, said assembly having aspects similar to aspects disclosed in FIGs 9A-9F but for septum connector cantilever 2004 and/or bracketing ribs 2009 and/or tab 2007 having rounded edges 2003 to provide access for engaging and disengaging cannula connector 1900. Connector 1900 has shroud 1901 and aperture 1904 accessing cavity 1903a for receiving connector 2000 and tab 2007 of cantilever 2004. Septum 109 as depicted in FIG. 2B, has surface 113 that slightly protrudes from the plane parallel to axis B for providing an unobstructed (not recessed) surface for swabbing with disinfectant prior to use.

In contrast to commercially available connector assemblies, FIGs. 7 thru FIGs. 20F provide for release from the locking configuration of the assembled connectors directly or indirectly via the cantilever feature of the septum connector. For example, the Interlink™ connector is configured to engage and disengage the septum connector by way of features configured on the cannula connector.

The cannula connector and or the septum connector components can be made from conventional thermoplastics suitable for injection molding, such as ABS, COC's, polycarbonates, PEEK, nylon, and the like. The septum can be made separately of a thermoset such as polyisoprene, polysilicone, polyisocynates, or natural rubber, or can be co-injected molded with a thermoplastic elastomer such as, Kraton™, Santoprene™, SBR, RIM polysilicones or polyisocynates, or the like. Adhesives may be employed to substantially join alternate attachments to the described embodiments, particularly attachments that may be coupled with luer connections, where applicable. Adhesives may be but are not limited to: cyanoacrylate, 2-part epoxy, heat-activated resin, UV cured adhesive and hot melt. Joining may also be achieved through, but not limited to, the use of solvent bonding, ultrasonics, spin welding, and heat-staking methods. All of the proposed aspects, or part thereof can be injection molded. Design intent may be such that designs are molded with simple open/close tooling to reduce tool cost and cycle times. Where connector features are not effectively produced by single tool molding; ultrasonic welding, heat forming, adhesives or mechanical retention may be employed to join one or more or all components, or to form new component features post molding. Furthermore, where dissimilar materials may be advantageous, a two-shot molding technique may be utilized.

While this disclosure is susceptible to embodiment in different forms, there are shown in the drawings and herein described in detail various embodiments of the disclosure. It should be understood, however, that the present disclosure is to be considered as an exemplification of the principles of the disclosure and is not intended to limit the disclosure to the embodiments illustrated. The scope of the invention is defined by the claims.

## Claims

1. A cannula connector (600, 700, 800, 1000, 1200, 1900) for establishing a locking connection with a septum connector (606, 706, 806), the cannula connector (600, 700, 800, 1000, 1200, 1900) comprising:
an access member (125, 625) having a generally annular flange disposed on one end and terminating in a cannula (112), the access member (125, 625) providing a generally cylindrical fluid passage between the annular flange and the cannula (112); and
a shroud (101, 201, 301, 401, 501, 601, 801, 1001, 1201, 1901) comprising a first wall (621) projecting from the access member (125, 625) between the annular flange and the cannula (112), the first wall (621) at least partially centrally surrounding the cannula (112) forming an opening sized to receive the septum portion (109) of the septum connector (606, 706, 806); **characterised in that** the cannula connector (600, 700, 800, 1000, 1200, 1900) further comprises:
a second wall and a third wall connecting a fourth wall, at least a portion of the fourth wall distally positioned from the first wall (621) so as to provide a cavity (603a, 803a, 1003a, 1203a, 1903a) in the opening; and
at least one aperture (304, 604, 704, 804, 1104, 1204, 1904) formed through the fourth wall.

2. The cannula connector (600, 700, 800, 1000, 1200, 1900) of claim 1,
wherein the cavity (603a, 803a, 1003a, 1203a, 1903a) is generally tapered towards the annular flange; or
wherein the second and third walls are generally parallel along at least a section thereof; or wherein at least a portion of the first wall (621) forms an opening about the cannula (112) that is generally cylindrical, the first wall (621) terminating in a generally annular edge (629); or
wherein at least a portion of the cavity (603a, 803a, 1003a, 1203a, 1903a) formed by the second, third, and fourth wall is generally rectangular, arched, or square; or
wherein at least a portion of the first wall (621) terminates in a generally annular edge (629) extending axially past the cannula (112).

3. The cannula connector (600, 700, 800, 1000, 1200, 1900) of claim 1, wherein the annular edge (629) comprises a cut out sized to accommodate a Y- or T- septum connector portion (806, 900, 1106, 1500, 1600), the cut out positioned generally opposed to the aperture (304, 604, 704, 804, 1104, 1204, 1904).

4. The cannula connector (600, 700, 800, 1000, 1200, 1900) of claim 1,
wherein the aperture (304, 604, 704, 804, 1104, 1204, 1904) is sized to receive a protrusion (203, 302, 404, 607, 707, 807, 907, 1007, 1107, 1307, 1507, 1607, 2007) from the septum connector (606, 706, 806) for providing a locking engagement; or
wherein the cannula (112) is blunt; or
wherein the cannula (112) comprises at least one side port opening.

5. The cannula connector (600, 700, 800, 1000, 1200, 1900) of claim 1, wherein the access member (125, 625) is a female luer.

6. A septum connector (606, 706, 806, 1600) for establishing a locking configuration with the cannula connector (600, 700, 800, 1000, 1200, 1900) of any one of claims 1-5, the septum connector (606, 706, 806) comprising:
a tubular housing (111) comprising a first end and a second end along a longitudinal axis (A) providing a generally cylindrical fluid passage between the first end and the second end; and
a penetrable septum (109) disposed on the first end and sealing the first end; **characterised in that** the septum connector (606, 706, 806, 1600) further comprises:
a cantilever (403, 605, 705, 884, 904, 1044, 1104, 1504, 1604, 2004) coupled to the housing (111) and extending axially generally along the longitudinal axis (A) of the tubular housing (111), the cantilever (403, 605, 705, 884, 904, 1044, 1104, 1504, 1604, 2004) configured to deflect generally normal to the longitudinal axis (A) of the housing (111), the cantilever (403, 605, 705, 884, 904, 1044, 1104, 1504, 1604, 2004) configured to engage with the cannula connector (600, 700, 800, 1000, 1200, 1900).

7. The septum connector (606, 706, 806, 1600) of claim 6, wherein the penetrable septum (109) is a spilt septum.

8. The septum connector (606, 706, 806, 1600) of claim 6, wherein the cantilever (403, 605, 705, 884, 904, 1044, 1104, 1504, 1604, 2004) resiliently deflects.

9. The septum connector (606, 706, 806, 1600) of claim 6, wherein the cantilever (403, 605, 705, 884, 904, 1044, 1104, 1504, 1604, 2004):
is coupled proximal to the penetrable septum (109) at one end, the terminus (1004a) of the cantilever (403, 605, 705, 884, 904, 1044, 1104, 1504, 1604, 2004) axially projecting generally away from the penetrable septum (109); or
is coupled at one end distally from the penetrable septum (109), the terminus (1004a) of the cantilever (403, 605, 705, 884, 904, 1044, 1104, 1504, 1604, 2004) axially projecting generally towards the penetrable septum (109); or
comprises a first surface distal to the housing (111) and a second surface proximal to the housing (111), the first and second surfaces separated by corresponding opposing edges; or
comprises at least one of protrusion (203, 302, 404, 607, 707, 807, 907, 1007, 1107, 1307, 1507, 1607, 2007) outwardly projecting from the first surface, the protrusion (203, 302, 404, 607, 707, 807, 907, 1007, 1107, 1307, 1507, 1607, 2007) configured to engage an aperture (304, 604, 704, 804, 1104, 1204, 1904) of the cannula connector (600, 700, 800, 1000, 1200, 1900) for locking therewith; or
comprises at least one protrusion (203, 302, 404, 607, 707, 807, 907, 1007, 1107, 1307, 1507, 1607, 2007) proximal to the penetrable septum (109) and inwardly projecting from the second surface, the protrusion (203, 302, 404, 607, 707, 807, 907, 1007, 1107, 1307, 1507, 1607, 2007) configured to engage a rim (110, 402) of the cannula connector (600, 700, 800, 1000, 1200, 1900) for locking therewith.

10. The septum connector (606, 706, 806, 1600) of claim 6, further comprising at pair of outwardly projecting walls axially positioned along the tubular housing (111) and generally adjacent the opposing edges of the cantilever (403, 605, 705, 884, 904, 1044, 1104, 1504, 1604, 2004).

11. The septum connector (606, 706, 806, 1600) of claim 6,
further comprising a male luer (107) coupled to the second end; or
further comprising an annular housing (206a, 1700) axially extending from the second end of the tubular housing (111), the inner circumferential surface of the annular housing having threading for engaging annular flanges; optionally, wherein the annular housing (206a, 1700) is rotatable about the longitudinal axis (A) of the tubular housing (111).

12. The septum connector (606, 706, 806, 1600) of any one of claims 6-11, wherein the penetrable septum (109) has an exposed upper surface extending beyond the first end of the tubular housing (111) along the longitudinal axis (A), the upper surface being configured and shaped for disinfection by wiping; or wherein the upper surface is flush with the first end.

13. The septum connector (606, 706, 806, 1600) of claim 12, wherein the upper surface is flush with the first end.

14. The septum connector (606, 706, 806, 1600) of claim 12, wherein the tubular housing (111) comprises a third end having a third fluid passage in communication with the fluid passage between the first end and the second end providing a Y- or T- septum connector (806, 900, 1106, 1500, 1600).

15. A connector assembly comprising the cannula connector (600, 700, 800, 1000, 1200, 1900) as defined in any one of claims 1-5, and the septum connector (606, 706, 806, 1600) as defined in any one of claims 6-14.

## Patentansprüche

1. Kanülenkonnektor (600, 700, 800, 1000, 1200, 1900) zum Herstellen einer Arretierverbindung mit einem Septumkonnektor (606, 706, 806), wobei der Kanülenkonnektor (600, 700, 800, 1000, 1200, 1900) umfasst:
ein Zugangselement (125, 625) mit einem allgemein kranzförmigen Flansch, der an einem Ende angeordnet ist und in einer Kanüle (112) endet, wobei das Zugangselement (125, 625) einen allgemein zylindrischen Fluiddurchgang zwischen dem kranzförmigen Flansch und der Kanüle (112) bereitstellt; und
eine Ummantelung (101, 201, 301, 401, 501, 601, 801, 1001, 1201, 1901), umfassend eine erste Wand (621), die aus dem Zugangselement (125, 625) zwischen dem kranzförmigen Flansch und der Kanüle (112) vorspringt, wobei die erste Wand (621) die Kanüle (112) mindestens teilweise zentral umgibt, wodurch eine Öffnung gebildet wird, die bemessen ist, um den Septumanteil (109) des Septumkonnektors (606, 706, 806) anzunehmen;
**dadurch gekennzeichnet, dass** der Kanülenkonnektor (600, 700, 800, 1000, 1200, 1900) des Weiteren umfasst:
eine zweite Wand und eine dritte Wand, welche mit einer vierten Wand verbunden ist, wobei mindestens ein Anteil der vierten Wand distal zu der ersten Wand (621) positioniert ist, um so einen Hohlraum (603a, 803a, 1003a, 1203a, 1903a) in der Öffnung bereitzustellen; und
mindestens einen Durchlass (304, 604, 704, 804, 1104, 1204, 1904), der durch die vierte Wand hindurch gebildet ist.

2. Kanülenkonnektor (600, 700, 800, 1000, 1200, 1900) nach Anspruch 1,
wobei der Hohlraum (603a, 803a, 1003a, 1203a, 1903a) allgemein in Richtung des kranzförmigen Flansches zuläuft; oder
wobei die zweite und dritte Wand allgemein entlang mindestens eines Abschnitts davon parallel sind; oder wobei mindestens ein Anteil der ersten Wand (621) eine Öffnung um die Kanüle (112) bildet, die allgemein zylindrisch ist, wobei die erste Wand (621) in einem allgemein kranzförmigen Rand (629) endet; oder
wobei mindestens ein Anteil des Hohlraums (603a, 803a, 1003a, 1203a, 1903a), der durch die zweite, dritte und vierte Wand gebildet ist, allgemein rechteckig, bogenförmig oder quadratisch ist; oder
wobei mindestens ein Anteil der ersten Wand (621) in einem allgemein kranzförmigen Rand (629) endet, der sich axial an der Kanüle (112) vorbei erstreckt.

3. Kanülenkonnektor (600, 700, 800, 1000, 1200, 1900) nach Anspruch 1, wobei der kranzförmige Rand (629) einen Ausschnitt umfasst, der bemessen ist, um einen Y- oder T-Septumkonnektoranteil (806, 900, 1106, 1500, 1600) unterzubringen, wobei der Ausschnitt allgemein gegenüber dem Durchlass (304, 604, 704, 804, 1104, 1204, 1904) positioniert ist.

4. Kanülenkonnektor (600, 700, 800, 1000, 1200, 1900) nach Anspruch 1,
wobei der Durchlass (304, 604, 704, 804, 1104, 1204, 1904) bemessen ist, um einen Vorsprung (203, 302, 404, 607, 707, 807, 907, 1007, 1107, 1307, 1507, 1607, 2007) von dem Septumkonnektor (606, 706, 806) anzunehmen, um einen Arretiereingriff bereitzustellen; oder
wobei die Kanüle (112) stumpf ist; oder
wobei die Kanüle (112) mindestens eine Seitenport-öffnung umfasst.

5. Kanülenkonnektor (600, 700, 800, 1000, 1200, 1900) nach Anspruch 1, wobei das Zugangselement (125, 625) ein weiblicher Luer ist.

6. Septumkonnektor (606, 706, 806, 1600) zur Herstellung einer Arretierkonfiguration mit dem Kanülenkonnektor (600, 700, 800, 1000, 1200, 1900) nach einem der Ansprüche 1 bis 5, wobei der Septumkonnektor (606, 706, 806) umfasst:
ein rohrförmiges Gehäuse (111), das ein erstes Ende und
ein zweites Ende entlang einer Längsachse (A) umfasst und einen allgemein zylindrischen Fluiddurchgang zwischen dem ersten Ende und dem zweiten Ende bereitstellt; und
ein durchdringbares Septum (109), das auf dem ersten Ende angeordnet ist und das erste Ende abdichtet;
**dadurch gekennzeichnet, dass** der Septumkonnektor (606, 706, 806, 1600) des Weiteren umfasst:
einen Ausleger (403, 605, 705, 884, 904, 1044, 1104, 1504, 1604, 2004), der an das Gehäuse (111) gekoppelt ist und sich axial allgemein entlang der Längsachse (A) des rohrförmigen Gehäuses (111) erstreckt, wobei der Ausleger (403, 605, 705, 884, 904, 1044, 1104, 1504, 1604, 2004) ausgestaltet ist, um allgemein normal zu der Längsachse (A) des Gehäuses (111) einzufedern, wobei der Ausleger (403, 605, 705, 884, 904, 1044, 1104, 1504, 1604, 2004) ausgestaltet ist, um in Eingriff mit dem Kanülenkonnektor (600, 700, 800, 1000, 1200, 1900) zu kommen.

7. Septumkonnektor (606, 706, 806, 1600) nach Anspruch 6, wobei das durchdringbare Septum (109) ein geteiltes Septum ist.

8. Septumkonnektor (606, 706, 806, 1600) nach Anspruch 6, wobei der Ausleger (403, 605, 705, 884, 904, 1044, 1104, 1504, 1604, 2004) elastisch eingefedert wird.

9. Septumkonnektor (606, 706, 806, 1600) nach Anspruch 6, wobei der Ausleger (403, 605, 705, 884, 904, 1044, 1104, 1504, 1604, 2004):
proximal an einem Ende an das durchdringbare Septum (109) gekoppelt ist, wobei der Endpunkt (1004a) des Auslegers (403, 605, 705, 884, 904, 1044, 1104, 1504, 1604, 2004) axial allgemein von dem durchdringbaren Septum (109) weg vorspringt; oder
an einem Ende distal von dem durchdringbare Septum (109) gekoppelt ist, wobei der Endpunkt (1004a) des Auslegers (403, 605, 705, 884, 904, 1044, 1104, 1504, 1604, 2004) axial allgemein in Richtung des durchdringbaren Septums (109) vorspringt; oder
eine erste Oberfläche distal des Gehäuses (111) und eine zweite Oberfläche proximal des Gehäuses (111) umfasst, wobei die erste und die zweite Oberfläche durch entsprechende gegenüberliegende Ränder getrennt sind; oder
mindestens einen von einem Vorsprung (203, 302, 404, 607, 707, 807, 907, 1007, 1107, 1307, 1507, 1607, 2007) umfasst, der von der ersten Oberfläche auswärts vorspringt, wobei der Vorsprung (203, 302, 404, 607, 707, 807, 907, 1007, 1107, 1307, 1507, 1607, 2007) ausgestaltet ist, um in Eingriff mit einem Durchlass (304, 604, 704, 804, 1104, 1204, 1904) des Kanülenkonnektors (600, 700, 800, 1000, 1200, 1900) zu kommen, um damit zu arretieren; oder
mindestens einen Vorsprung (203, 302, 404, 607, 707, 807, 907, 1007, 1107, 1307, 1507, 1607, 2007) proximal zu dem durchdringbaren Septum (109) umfasst, und der von der zweiten Oberfläche einwärts vorspringt, wobei der Vorsprung (203, 302, 404, 607, 707, 807, 907, 1007, 1107, 1307, 1507, 1607, 2007) ausgestaltet ist, um in Eingriff mit einer Einfassung (110, 402) des Kanülenkonnektors (600, 700, 800, 1000, 1200, 1900) zu kommen, um damit zu arretieren.

10. Septumkonnektor (606, 706, 806, 1600) nach Anspruch 6, des Weiteren umfassend ein Paar auswärts vorspringender Wände, die axial entlang des rohrförmigen Gehäuses (111) und allgemein benachbart zu den gegenüberliegenden Rändern des Auslegers (403, 605, 705, 884, 904, 1044, 1104, 1504, 1604, 2004) positioniert sind.

11. Septumkonnektor (606, 706, 806, 1600) nach Anspruch 6,
des Weiteren umfassend einen männlichen Luer (107), der an das zweite Ende gekoppelt ist; oder
des Weiteren umfassend ein kranzförmiges Gehäuse (206a, 1700), das sich axial von dem zweiten Ende des rohrförmigen Gehäuses (111) erstreckt, wobei die innere Umfangoberfläche des kranzförmigen Gehäuses ein Gewinde aufweist, um in Eingriff mit kranzförmigen Flanschen zu kommen; wobei das kranzförmige Gehäuse (206a, 1700) um die Längsachse (A) des rohrförmigen Gehäuses (111) drehbar ist.

12. Septumkonnektor (606, 706, 806, 1600) nach einem der Ansprüche 6 bis 11, wobei das durchdringbare Septum (109) eine exponierte Oberseite aufweist, die sich entlang der Längsachse (A) über das erste Ende des rohrförmigen Gehäuses (111) hinaus erstreckt, wobei die Oberseite zur Desinfektion durch Wischen ausgestaltet und geformt ist; oder wobei die Oberseite bündig mit dem ersten Ende ist.

13. Septumkonnektor (606, 706, 806, 1600) nach Anspruch 12, wobei die Oberseite mit dem ersten Ende bündig ist.

14. Septumkonnektor (606, 706, 806, 1600) nach Anspruch 12, wobei das rohrförmige Gehäuse (111) ein drittes Ende mit einem dritten Fluiddurchgang in Kommunikation mit dem Fluiddurchgang zwischen dem ersten Ende und dem zweiten Ende umfasst, wodurch ein Y- oder T-Septumkonnektor (806, 900, 1106, 1500, 1600) bereitgestellt wird.

15. Konnektorbaugruppe, umfassend den Kanülenkonnektor (600, 700, 800, 1000, 1200, 1900), wie in einem der Ansprüche 1 bis 5 definiert, und den Septumkonnektor (606, 706, 806, 1600), wie in einem der Ansprüche 6 bis 14 definiert.

## Revendications

1. Raccord de canule (600, 700, 800, 1000, 1200, 1900) pour établir un raccord de verrouillage avec un raccord de cloison (606, 706, 806), le raccord de canule (600, 700, 800, 1000, 1200, 1900) comprenant :
un élément d'accès (125, 625) ayant une bride généralement annulaire disposée au niveau d'une extrémité et se terminant par une canule (112), l'élément d'accès (125, 625) fournissant un passage de fluide généralement cylindrique entre la bride annulaire et la canule (112) ; et
une enveloppe (101, 201, 301, 401, 501, 601, 801, 1001, 1201, 1901) comprenant une première paroi (621) faisant saillie depuis l'élément d'accès (125, 625) entre la bride annulaire et la canule (112), la première paroi (621) entourant au moins partiellement au centre la canule (112) formant une ouverture dimensionnée pour recevoir la partie cloison (109) du raccord de cloison (606, 706, 806) ;
**caractérisé en ce que** le raccord de canule (600, 700, 800, 1000, 1200, 1900) comprend en outre :
une deuxième paroi et une troisième paroi raccordant une quatrième paroi, au moins une partie de la quatrième paroi étant positionnée de manière distale par rapport à la première paroi (621) de façon à fournir une cavité (603a, 803a, 1003a, 1203a, 1903a) dans l'ouverture ; et
au moins une ouverture (304, 604, 704, 804, 1104, 1204,
1904) formée à travers la quatrième paroi.

2. Raccord de canule (600, 700, 800, 1000, 1200, 1900) selon la revendication 1,
la cavité (603a, 803a, 1003a, 1203a, 1903a) étant généralement effilée vers la bride annulaire ; ou
les deuxième et troisième parois étant généralement parallèles le long d'au moins une section de celles-ci, ou
au moins une partie de la première paroi (621) formant une ouverture autour de la canule (112) qui est généralement cylindrique, la première paroi (621) se terminant par un bord généralement annulaire (629), ou au moins une partie de la cavité (603a, 803a, 1003a, 1203a, 1903a) formée par les deuxième, troisième et quatrième parois étant généralement rectangulaire, arquée ou carrée ; ou
au moins une partie de la première paroi (621) se terminant par un bord généralement annulaire (629) s'étendant axialement au-delà de la canule (112).

3. Raccord de canule (600, 700, 800, 1000, 1200, 1900) selon la revendication 1, le bord annulaire (629) comprenant une découpe dimensionnée pour recevoir une partie de raccord de cloison en Y ou en T (806, 900, 1106, 1500, 1600), la découpe étant positionnée généralement à l'opposé de l'ouverture (304, 604, 704, 804, 1104, 1204, 1904) .

4. Raccord de canule (600, 700, 800, 1000, 1200, 1900) selon la revendication 1,
l'ouverture (304, 604, 704, 804, 1104, 1204, 1904) étant dimensionnée pour recevoir une partie saillante (203, 302, 404, 607, 707, 807, 907, 1007, 1107, 1307, 1507, 1607, 2007) depuis le raccord de cloison (606, 706, 806) pour fournir une mise en prise de verrouillage ; ou
la canule (112) étant émoussée ; ou
la canule (112) comportant au moins une ouverture d'orifice latérale.

5. Raccord de canule (600, 700, 800, 1000, 1200, 1900) selon la revendication 1, l'élément d'accès (125, 625) étant un luer femelle.

6. Raccord de cloison (606, 706, 806, 1600) pour établir une configuration de verrouillage avec le raccord de canule (600, 700, 800, 1000, 1200, 1900) selon l'une quelconque des revendications 1 à 5, le raccord de cloison (606, 706, 806) comprenant :
un boîtier tubulaire (111) comprenant une première extrémité et une deuxième extrémité le long d'un axe longitudinal (A) fournissant un passage de fluide généralement cylindrique entre la première extrémité et la deuxième extrémité ; et
une cloison pénétrable (109) disposée sur la première extrémité et étanchéifiant la première extrémité ;
**caractérisé en ce que** le raccord de cloison (606, 706, 806, 1600) comprend en outre :
une extension (403, 605, 705, 884, 904, 1044, 1104, 1504, 1604, 2004) couplée au boîtier (111) et s'étendant axialement généralement le long de l'axe longitudinal (A) du boîtier tubulaire (111), l'extension (403, 605, 705, 884, 904, 1044, 1104, 1504, 1604, 2004) étant configurée pour dévier de façon généralement normale par rapport à l'axe longitudinal (A) du boîtier (111), l'extension (403, 605, 705, 884, 904, 1044, 1104, 1504, 1604, 2004) étant configurée pour venir en prise avec le raccord de canule (600, 700, 800, 1000, 1200, 1900).

7. Raccord de cloison (606, 706, 806, 1600) selon la revendication 6, la cloison pénétrable (109) étant une cloison renversée.

8. Raccord de cloison (606, 706, 806, 1600) selon la revendication 6, l'extension (403, 605, 705, 884, 904, 1044, 1104, 1504, 1604, 2004) déviant de manière élastique.

9. Raccord de cloison (606, 706, 806, 1600) selon la revendication 6, l'extension (403, 605, 705, 884, 904, 1044, 1104, 1504, 1604, 2004) :
étant couplée de manière proximale par rapport à la cloison pénétrable (109) au niveau d'une extrémité, la terminaison (1004a) de l'extension (403, 605, 705, 884, 904, 1044, 1104, 1504, 1604, 2004) faisant axialement saillie généralement à l'opposé de la cloison pénétrable (109), ou
étant couplé au niveau d'une extrémité de manière distale par rapport à la cloison pénétrable (109), la terminaison (1004a) de l'extension (403, 605, 705, 884, 904, 1044, 1104, 1504, 1604, 2004) faisant axialement saillie généralement vers la cloison pénétrable (109), ou
comprenant une première surface distale par rapport au boîtier (111) et une deuxième surface proximale par rapport au boîtier (111), la première et la deuxième surface étant séparées par des bords opposés correspondants ; ou
comprenant au moins une partie saillante (203, 302, 404, 607, 707, 807, 907, 1007, 1107, 1307, 1507, 1607, 2007) faisant saillie vers l'extérieur depuis la première surface, la partie saillante (203, 302, 404, 607, 707, 807, 907, 1007, 1107, 1307, 1507, 1607, 2007) étant configurée pour venir en prise avec une ouverture (304, 604, 704, 804, 1104, 1204, 1904) du raccord de canule (600, 700, 800, 1000, 1200, 1900) pour se verrouiller avec celle-ci ; ou
comprenant au moins une partie saillante (203, 302, 404, 607, 707, 807, 907, 1007, 1107, 1307, 1507, 1607, 2007) de manière proximale par rapport à la cloison pénétrable (109) et faisant saillie vers l'intérieur depuis la deuxième surface, la partie saillante (203, 302, 404, 607, 707, 807, 907, 1007, 1107, 1307, 1507, 1607, 2007) étant configurée pour venir en prise avec un rebord (110, 402) du raccord de canule (600, 700, 800, 1000, 1200, 1900) pour se verrouiller avec celui-ci.

10. Raccord de cloison (606, 706, 806, 1600) selon la revendication 6, comprenant en outre une paire de parois faisant saillie vers l'extérieur, positionnées axialement le long du boîtier tubulaire (111) et généralement adjacentes aux bords opposés de l'extension (403, 605, 705, 884, 904, 1044, 1104, 1504, 1604, 2004).

11. Raccord de cloison (606, 706, 806, 1600) selon la revendication 6,
comprenant en outre un luer mâle (107) couplé à la deuxième extrémité ; ou
comprenant en outre un boîtier annulaire (206a, 1700) s'étendant axialement depuis la deuxième extrémité du boîtier tubulaire (111), la surface circonférentielle interne du boîtier annulaire ayant un filetage pour venir en prise avec des brides annulaires ; éventuellement, le boîtier annulaire (206a, 1700) pouvant tourner autour de l'axe longitudinal (A) du boîtier tubulaire (111).

12. Raccord de cloison (606, 706, 806, 1600) selon l'une quelconque des revendications 6 à 11, la cloison pénétrable (109) ayant une surface supérieure exposée s'étendant au-delà de la première extrémité du boîtier tubulaire (111) le long de l'axe longitudinal (A), la surface supérieure étant configurée et formée pour une désinfection par essuyage ; ou la surface supérieure étant au même niveau que la première extrémité.

13. Raccord de cloison (606, 706, 806, 1600) selon la revendication 12, la surface supérieure étant au même niveau que la première extrémité.

14. Raccord de cloison (606, 706, 806, 1600) selon la revendication 12, le boîtier tubulaire (111) comprenant une troisième extrémité ayant un troisième passage de fluide en communication avec le passage de fluide entre la première extrémité et la deuxième extrémité fournissant un raccord de cloison en Y ou en T (806, 900, 1106, 1500, 1600).

15. Ensemble raccord comprenant le raccord de canule (600, 700, 800, 1000, 1200, 1900) selon l'une quelconque des revendications 1 à 5, et le raccord de cloison (606, 706, 806, 1600) selon l'une quelconque des revendications 6 à 14.
